# EUROPEAN PATENT APPLICATION

(11) **EP 4 265 603 A1**
(43) Date of publication of application: **25.10.2023**
(21) Application number: 22774074.3
(22) Date of filing: 14.03.2022
(51) Int. Cl.: C07D 307/94, C07D 307/00, A61K 31/34, A61P 25/00

(54) **AGAROFURAN COMPOUND, PREPARATION METHOD THEREFOR, AND APPLICATION THEREOF**

(30) Priority: 23.03.2021 CN 202110305886
(71) Applicant: Beijing Chy Pharmaceutical Technology Co. Ltd., Beijing 102600 (CN)
(72) Inventor: CHENG, Xiang, Beijing 102600 (CN)
(74) Representative: LLR
(86) International application number: PCT/CN2022/080670
(87) International publication number: WO 2022/199412

(57) **Abstract**

Embodiments of the present application relate to an agarofuran compound, a preparation method therefor, and an application thereof. In the embodiments of the present application, structural modification is performed on main metabolic sites, such as alkyl side chain, of 4-butyl-α-agarofuran (BAF) to provide a novel agarofuran compound having higher activity, a better therapeutic effect, and better drug metabolism properties to treat mental illnesses such as anxiety disorders.

## Description

### Cross-Reference to Related Application

The present application claims priority to Chinese patent application No. 202110305886.0, filed with the Chinese Patent Office, and entitled "Agarofuran Compound, Preparation Method therefor, and Application Thereof", the entire contents of which are incorporated herein by reference.

### Technical Field

The present application relates to the technical field of biomedicines, in particular to an agarofuran-based compound, a preparation method therefor, and an application thereof.

### Background Art

Anxiety is a human central nervous system disease or disorder, and with the daily increases of human social life, work rhythm and pressure, the incidence rate of the anxiety disorder is increased day by day.

It is indicated from a large number of clinical epidemiological investigation results on mental diseases that comorbidities with mental diseases are very common, and among different types of the comorbidities, the most common one is actually the anxiety disorder that is comorbid with the depression disorder. It is indicated from a large number of researches that the comorbidity anxiety disorder may increase the severity of existing depression, reduce the recovery rate, increase the readmission rate, increase the suicide risk, and decrease social and occupational functions. Similar trends also exist in patients with other psychiatric comorbidity anxiety disorder patients. Therefore, the treatment of the anxiety disorder is particularly important. At present, the treatment for the anxiety disorder is insufficient worldwide, only 9.8% receives probably sufficient treatment, and among anxiety disorder patients, only 41.3% perceives treatment needs, and the corresponding proportions of Chinese population are lower than the global average.

On the one hand, the treatment of the anxiety disorder is particularly important, and on the other hand, the treatment is insufficient, this situation makes us ponder that there is still a long way to go to improve the standardized sufficient treatment of the anxiety disorder. At present, there are far fewer types of clinically specific drugs for treating the anxiety disorder than antidepressants. So for the mental disorder, treating anxiety is a key to treatment.

Among antianxiety drugs developed already, the representative drug is a benzodiazepine compound, but in clinical treatment, it is discovered that the benzodiazepine compound has side effects such as tolerance, dependence, relapse after drug withdrawal or delayed efficacy. Therefore, it is very necessary to develop an antianxiety drug with better efficacy and fewer side effects. Agarwood is a traditional precious Chinese medicinal herb that contains a plurality of agarofuran-based compounds, and the agarofuran-based drug 4-butyl-α-agarofuran (BAF) is a known novel antianxiety drug, which is already approved for clinical Phase II\III permission at present. This compound has significant antianxiety activity, and its side effects are extremely minimal, thus few adverse events occur. However, this drug has worse pharmacokinetic properties, specifically manifested in low total exposure dose, low bioavailability, and short half-life and the like, so the efficacy is affected to some extent

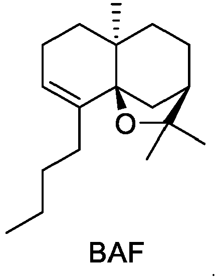

The information disclosed in the background art part is only intended to increase the understanding of the overall background of the present application, and should not be regarded as acknowledging or implying in any forms that the information constitutes the prior art that is already known to those of ordinary skill in the art.

### Summary of the Invention

### Purpose of Application

The purpose of the present application is to provide a novel agarofuran-based compound with higher activity, better efficacy, and better pharmacokinetic properties for treatment of mental diseases such as an anxiety disorder by structural modifications of main metabolic sites such as alkyl side chains of 4-butyl-α-agarofuran (BAF).

### Solution Scheme

In order to achieve the purpose of the present application, embodiments of the present application provide the following technical solutions:

One aspect of the present application provides an agarofuran-based compound, and its structure is shown in General formula (I): 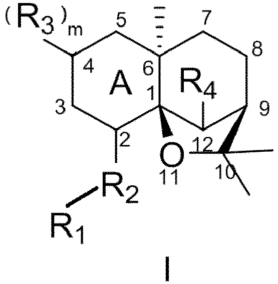 wherein there is no double bond in the ring A or there is one double bond located in the position 2-3 of the ring A;
R₁ is methyl that is monosubstituted, disubstituted, or trisubstituted by fluorine atom(s);
R₂ is C₂-C₉ linear hydrocarbylene, branched hydrocarbylene or cycloalkylene that is unsubstituted, hydroxyl-substituted or carbonyl-substituted, and the linear hydrocarbylene or branched hydrocarbylene is alkylene or alkenylene containing 1-3 double bonds;
m represents the number of R₃ groups linked to carbon in the position 4 of the ring A, m=1 or 2, and when m=1, R₃ is selected from hydrogen atom, carbonyl, or hydroxyl; when m=2, R₃ is deuterium atom; and
R₄ is selected from hydrogen atom, deuterium atom, carbonyl or hydroxyl.

As described above, the ring A represents an overall oxatricyclo, wherein: R₂ is linked in the position 2 of the ring A, R₃ is linked in the position 4 of the ring A, R₄ is linked in the position 12 of the ring A, and m represents the number of R₃ groups, rather than the number of repeating units in the same group.

In one possible embodiment of the above agarofuran-based compound shown in General formula (I), R₂ is C₂-C₅ linear alkylene that is unsubstituted, hydroxyl-substituted, or carbonyl-substituted.

In one possible embodiment of the above agarofuran-based compound shown in General formula (I), R₂ is C₃-C₄ linear alkylene that is unsubstituted, hydroxyl-substituted, or carbonyl-substituted. In one possible embodiment of the above agarofuran-based compound shown in General formula (I), R₂ is C₃ linear alkylene that is unsubstituted, hydroxyl-substituted, or carbony-substituted.

In one possible embodiment of the above agarofuran-based compound shown in General formula (I), it is selected from the following compounds:

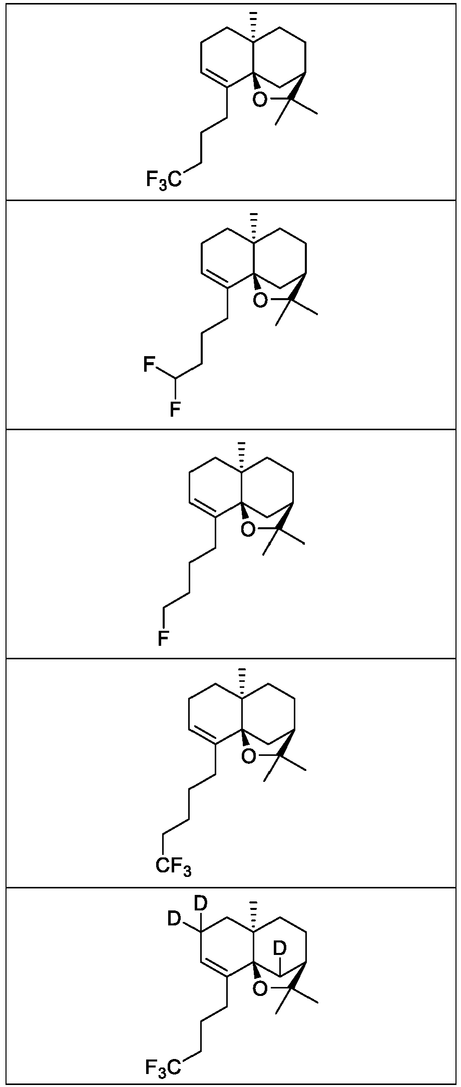

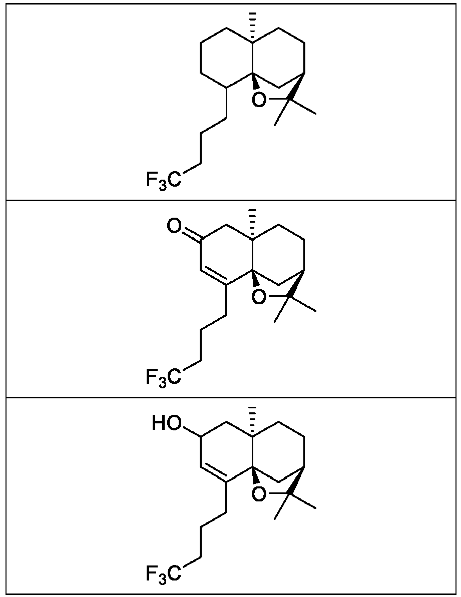

Another aspect of the present application relates to a pharmaceutical composition, and it contains an agarofuran-based compound shown in General formula (I), a pharmaceutically acceptable salt thereof, or a prodrug thereof, and also contains at least one of a pharmaceutically acceptable carrier, a diluent, or an excipient.

Another aspect of the present application relates to a use of the agarofuran-based compound shown in General formula (I), the pharmaceutically acceptable salt thereof, the prodrug thereof, or the pharmaceutical composition containing the same in preparation of a drug for preventing and/or treating an anxiety disorder.

Another aspect of the present application relates to a method for treating an anxiety disorder, including administering a therapeutically effective amount of the agarofuran-based compound shown in General formula (I), the pharmaceutically acceptable salt thereof, the prodrug thereof, or the pharmaceutical composition containing the same to a subject in need of treating an anxiety disorder.

The agarofuran-based compound shown in General formula (I) of the present application or the pharmaceutical composition containing the same may be administered in the form of unit dose, and the route of administration may be intestinal or non-intestinal, such as oral, muscular, subcutaneous, nasal, oral mucosa, skin, peritoneum or rectum. The dosage form, such as tablets, capsules, drop pills, aerosols, pills, powders, solutions, suspensions, emulsions, granules, suppositories, and freeze-dried powder injections, may be an ordinary preparation, a sustained-release preparation, a controlled-release preparation, and various particle administration systems.

The administration dose of a drug depends on various factors, including but not limited to the following factors: activity of a specific compound used, nature and severity of a disease to be treated, patient's gender, age, weight, health status, behavior, diet, administration time, administration mode, excretion rate, drug combination and the like. In addition, the optimal treatment mode, such as a treatment mode, and the daily dose of the compound shown in General formula (I) may be verified according to traditional treatment plans.

Another aspect of the present application relates to a preparation method for the agarofuran-based compound shown in General formula (I), which may refer to a preparation method of "Stereoselective Synthesis of Antianxiety Candidate Drug AF-5 Using (+) Dihydrocarvone as Raw Material" (Yin Dali, et al., "Chinese Journal of Medicinal chemistry", 2003, Volume 13, Issue 4, Pages 187-193).The preparation method is selected from any one of the followings:
(1) When there is one double bond located in the position 2-3 of the ring A of the agarofuran-based compound shown in General formula (I), m=1, and R₃ and R₄ are both hydrogen atoms, the preparation method includes the following steps: 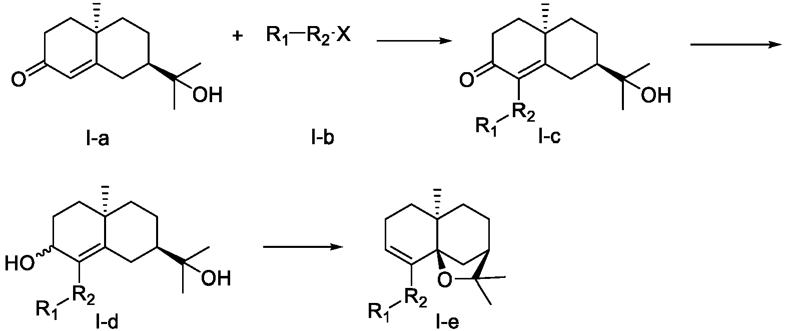
   wherein, compounds shown in Formulas (I-a) and (I-b) are subjected to a substitution reaction under an alkaline condition, to obtain a compound shown in Formula (I-c);R₁ is methyl that is monosubstituted, disubstituted, or trisubstituted by fluorine atom(s);R₂ is C₂-C₉ linear hydrocarbylene, branched hydrocarbylene or cycloalkylene that is unsubstituted, hydroxyl-substituted or carbonyl-substituted, and the linear hydrocarbylene or branched hydrocarbylene is alkylene or alkenylene containing 1-3 double bonds; X is halogen, preferably bromine;
   the compound shown in Formula (I-c) is subjected to a reduction reaction in the presence of a reducing agent, to obtain a compound shown in Formula (I-d); and
   the compound shown in Formula (I-d) is subjected to a cyclization reaction under an acidic condition, to obtain a compound shown in Formula (I-e).
(2) When there is no double bond in the ring A of the agarofuran-based compound shown in General formula (I), m=1, and R₃ and R₄ are both hydrogen, the preparation method includes the following steps: the compound (I-e) in the preparation method (1) is subjected to catalytic hydrogenation to obtain a compound shown in Formula (I-f); 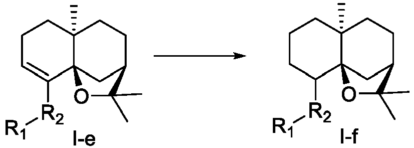
(3) When there is one double bond located in the position 2-3 of the ring A of the agarofuran-based compound shown in General formula (I), m=2, and R₃ and R₄ are both deuterium, the preparation method includes the following steps: 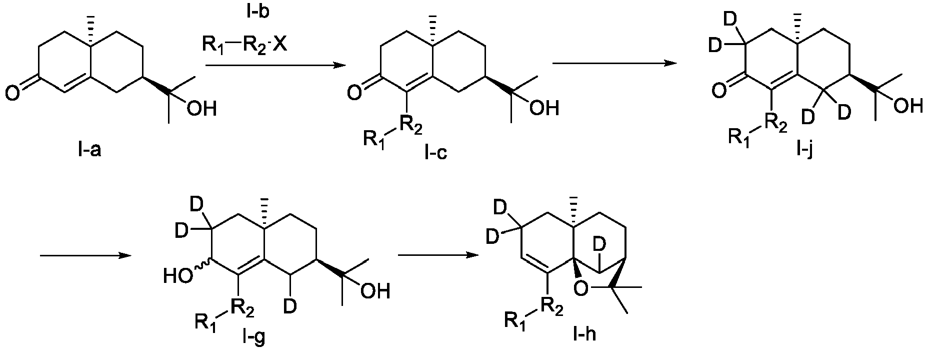
   wherein, compounds shown in Formulas (I-a) and (I-b) are subjected to a substitution reaction under an alkaline condition, to obtain a compound shown in Formula (I-c);R₁ is methyl that is monosubstituted, disubstituted, or trisubstituted by fluorine atom(s);R₂ is C₂-C₉ linear hydrocarbylene, branched hydrocarbylene or cycloalkylene that is unsubstituted, hydroxyl-substituted or carbonyl-substituted, and the linear hydrocarbylene or branched hydrocarbylene is alkylene or alkenylene containing 1-3 double bonds; X is halogen, preferably bromine;
   the compound shown in Formula (I-c) is subjected to a deuteration reaction with deuterated alcohol under an alkaline condition, to generate a compound shown in Formula (I-j);
   the compound shown in Formula (I-j) is subjected to a reduction reaction in the presence of a reducing agent, to obtain a compound shown in Formula (I-g);
   the compound shown in Formula (I-g) is subjected to a cyclization reaction under an acidic condition, to obtain a compound shown in Formula (I-h).
(4) When there is no double bond in the ring A of the agarofuran-based compound shown in General formula (I), m=2, and R₃ and R₄ are both deuterium, the preparation method includes the following steps: the compound (I-h) in the preparation method (3) is subjected to catalytic hydrogenation to obtain a compound shown in Formula (I-i); 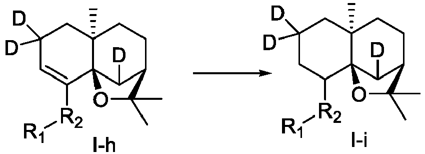
(5) When there is one double bond in the ring A of the agarofuran-based compound shown in General formula (I), m=1, R₃ is carbonyl, and R₄ is hydrogen, the preparation method includes the following steps: the compound in Formula (I-e) in the preparation method (1) is subjected to an oxidation reaction in the presence of an oxidant, to obtain a compound shown in Formula (I-k); 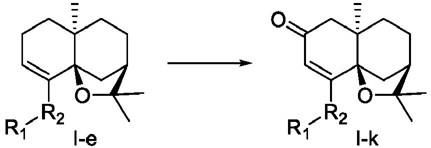
(6) When there is one double bond in the ring A of the agarofuran-based compound shown in General formula (I), m=1, R₃ is hydroxyl, and R₄ is hydrogen, the preparation method includes the following steps: the compound in Formula (I-k) in the preparation method (5) is subjected to a reduction reaction in the presence of a reducing agent, to obtain a compound shown in Formula (I-m);

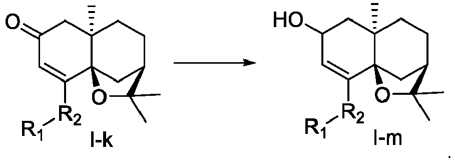

In one possible embodiment of the above preparation method, R₂ is C₂-C₅ linear alkylene that is unsubstituted, hydroxyl-substituted, or carbonyl-substituted; optionally, R₂ is C₃-C₄ linear alkylene that is unsubstituted, hydroxyl-substituted, or carbonyl-substituted; and further optionally, R₂ is -C₃ linear alkylene that is unsubstituted, hydroxyl-substituted, or carbonyl-substituted.

In one possible embodiment of the above preparation method, R₁ and R₂ are selected from any one of the following combinations:

1. R₁ = -CF₃, R₂ = -CH₂CH₂CH₂-

2. R₁ = -CHF₂, R₂ = -CH₂CH₂CH₂-

3. R₁ = -CH₂F, R₂ = -CH₂CH₂CH₂-

4. R₁ = -CF₃, R₂ = -CH₂CH₂CH₂CH₂-

In one possible embodiment of the above preparation method, the reagent for providing an alkaline condition mainly includes an inorganic base, and the inorganic base includes: one or more of sodium hydride, sodium hydroxide, potassium hydroxide, sodium tert-butanol, and potassium tert-butanol.

In one possible embodiment of the above preparation method, the reducing agent includes: one or more of sodium borohydride, potassium borohydride, and lithium aluminum hydride.

In one possible embodiment of the above preparation method, the reagent for providing an acidic condition includes: one or more of trifluoroacetic acid, hydrochloric acid, sulfuric acid, phosphoric acid and p-toluenesulfonic acid.

In one possible embodiment of the above preparation method, the metal catalyst for the catalytic hydrogenation includes: one or more of palladium carbon, rhodium carbon, platinum dioxide, and Raney nickel.

In one possible embodiment of the above preparation method, the oxidant for the oxidation reaction includes: one or more of oxygen, hydrogen peroxide, peroxide, and Sarrett reagent.

In one possible embodiment of the above preparation method, the solvents used in the preparation method (1) include: Step 1: one or more of methanol, ethanol, isopropanol, and tert-butanol; Step 2: one or more of ether and tetrahydrofuran; and Step 3: one or more of n-hexane and petroleum ether.

In one possible embodiment of the above preparation method, the solvent used in the preparation method (2) includes: one or more of ethanol, methanol, and ether.

In one possible embodiment of the above preparation method, the solvents used in the preparation method (3) include: Step 1: one or more of methanol, ethanol, isopropanol and tert-butanol; Step 2: one or more of deuterated methanol and deuterated ethanol; Step 3: one or more of ether and tetrahydrofuran; and Step 4: one or more of n-hexane and petroleum ether.

In one possible embodiment of the above preparation method, the solvent used in the preparation method (4) includes: one or more of ethanol, methanol, and ether.

In one possible embodiment of the above preparation method, the solvent used in the preparation method (5) includes: one or more of dichloromethane, toluene, benzene, and pyridine.

In one possible embodiment of the above preparation method, the solvent used in the preparation method (6) includes: one or more of ethanol, methanol, and ether.

### Beneficial Effect

The agarofuran-based compound shown in General formula (I) in the embodiments of the present application shows excellent antianxiety activity, and it has the characteristics of rapid effects and high safety factors compared with known anxiolytic benzodiazepine compounds (such as diazepam), and has the advantages of better pharmacokinetic properties, such as more suitable half-life, higher exposure dose, higher bioavailability and the like compared with 4-butyl-α-agarofuran.

### Brief Description of the Drawings

Fig. 1 shows the effect of drug intraperitoneal injection on open arm and closed arm residence times of elevated plus maze for mice in Example 8 (Mean ± SEM, n=13). Compared with the excipient, *P<0.05, **P<0.01, and ***P <0.001; and compared with a BAF (2 mg/kg) group, #P<0.05.
Fig. 2 shows the effect of drug intraperitoneal injection on open arm and closed arm entries of elevated plus maze for mice in Example 8 (Mean ± SEM, n=13). Compared with the excipient, *P<0.05, and **P<0.01.
Fig. 3 shows the effect of drug intraperitoneal injection on open arm and close arm entry ratios and time ratios of elevated plus maze in Example 8 (Mean ± SEM, n=13). Compared with the excipient,*P<0.05, and **P<0.01.
Fig. 4 shows a plasma drug-time curve of a prototype drug after intraperitoneal injection of BAF (10 mg/kg) into a mouse in Example 8.
Fig. 5 shows a plasma drug-time curve of a prototype drug after intraperitoneal injection of compound 2 (10 mg/kg) into a mouse in Example 8.

### Detailed Description of the Invention

In order to make purposes, technical solutions, and advantages of embodiments of the present application clearer, the technical solutions in the embodiments of the present application are clearly and completely described below. Apparently, the examples described are a part of the examples of the present application, not all of the examples. Based on the examples in the present application, all other examples obtained by those of ordinary skill in the art without creative labor shall fall within the scope of protection of the present application.

In addition, in order to describe the present application better, numerous specific details are provided in specific embodiments thereinafter. Those skilled in the art should understand that without certain specific details, the present application may also be implemented. In some examples, there is no detailed description of raw materials, elements, methods, means and the like that are familiar to those skilled in the art, as to highlight the main purpose of the present application.

Unless otherwise stated explicitly, in the entire description and claims, the term "including" or its transformations such as "containing" or "comprising" may be understood to include the stated elements or constituent parts, without excluding other elements or other constituent parts.

Unless otherwise stated, terms used in the description and claims have the following meanings: The term "alkylene" includes a linear or branched alkylene containing 2-9 carbon atoms, such as propylene, isopropylene, n-butylene, isobutylene, sec-butylene, tert-butylene, pentylene, isopentylene, neopentylene, and sec-pentylene, and the alkylene is unsubstituted or substituted by a hydroxyl or a carbonyl.

In the present application, the term "alkenylene containing 1-3 double bonds" refers to a linear or branched alkenylene with 1-3 double bonds, such as vinylene, propenylene, 3-enbutylene, 2-enbutylene, 2-enpentylene, and 3-enpentylene, and the alkenylene is unsubstituted or substituted by a hydroxyl or a carbonyl.

In the examples of the present application, the structures of the compounds are all determined by nuclear magnetic resonance (NMR) or/and mass spectrometry (MS). NMR is determined with a Varian Mercury-400 or Bruker-600 nuclear magnetic spectrometer, the solvent for determination is deuterated methanol (CD₃OD), deuterated chloroform (CDCl₃), and the internal standard is tetramethylsilane (TMS).

MS is determined with an ESI mass spectrometer (manufacturer: Thermo, and model: Exactive Plus).

High Performance Liquid Chromatography (HPLC) is determined by using an Agilent 1200 high-pressure liquid chromatograph (Cs 150×4.6 mm chromatographic analysis column).

A Qingdao GF254 silica gel plate is used for thin-layer chromatography, with a specification of 0.15-0.2 mm.

The column chromatography uses Yantai Huanghai 300-400-mesh silica gel as a carrier.

The known starting materials of the present application may be synthesized by using or following methods known in the art, or purchased from reagent companies such as ITC, Alfa, Bide, and Inno Chem. In the examples, unless otherwise specified, reactions may be performed under argon gas or nitrogen gas.

The reaction process in the examples is monitored by a thin-layer chromatography (TLC), a developing agent system used in the reaction is mainly petroleum ether and ethyl acetate, and the volume ratio of solvents is adjusted according to different polarities of the compounds.

An eluent system of the column chromatography used to purify the compound is mainly petroleum ether and ethyl acetate, and the volume ratio of solvents is adjusted according to different polarities of the compounds.

### Example 1:

### Step 1

### Preparation of (6R,9R)6-methyl-9-(1-hydroxyisopropyl)-2-(4,4,4-trifluorobutyl)dicyclo[4.4.0]deca-1-ene-3-one (1)

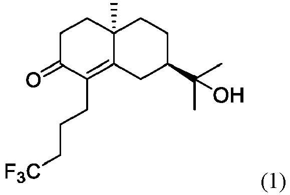

Under argon protection, a compound (1000 mg, and 4.50 mmol) shown in Formula (I-a) and KOH (343 mg, and 6.117 mmol) were dissolved in tert-butanol, and heated to reflux, 10 mL of 4-bromo-1,1,1-trifluorobutane (1051 mg, and 5.53 mmol) solution was dropwise added therein, and it was continued to react for 1 h after dropping. The reaction material was cooled, 10 mL of distilled water was added therein, then the material was neutralized with 1N hydrochloric acid until pH=7, and evaporated to remove the solvent, the residue after evaporation was dissolved in ether, an ether layer was washed with saturated sodium chloride, dried with anhydrous sodium sulfate, and evaporated to remove the solvent. A crude product was purified by silica gel column chromatography, and eluted with a mixed solvent of petroleum ether/ethyl acetate (10:1), to obtain a required compound 1 (745 mg, and yield: 64%). [α]_{D}²⁰ = -66.6° (c 0.89, CHCl3), ¹H NMR (600 MHz, CDCl₃) δ 1.21 (s, 3 H), 1.72 (s, 3 H), 1.75 (s, 3 H), 1.42-1.48 (m, 2 H), 1.50-1.56 (m, 2 H), 1.78-1.83 (m, 5 H), 1.91-1.98 (m, 2 H), 2.05-2.13 (m, 2 H), 2.33-2.37 (m, 1 H), 2.41-2.51 (m, 3 H), 2.58-2.64 (m, 1 H), 2.67-2.71 (m, 1 H). ¹³C NMR (150 MHz, CDCl₃) δ 198.1, 166.5, 133.2, 73.4, 44.0, 36.9, 36.4, 35.9, 34.1, 33.7, 33.5, 29.0, 27.1, 27.0, 25.0, 24.4, 21.5, 21.4, 21.2. HR-MS *m*/*z* (ESI) 333.2030 [M+H]⁺, C₁₈H₂₈O₂F₃ Calcd. for 333.2036.

### Step 2

### Preparation of (1R,6S,9R)6,10,10-trimethyl-2-(4,4,4-trifluorobutyl)-11-oxatricyclo[7.2.1.0^{1,6}]dodec-2-ene (2)

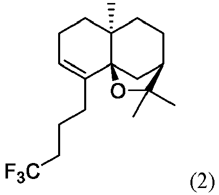

The compound 1 (530 mg, and 1.58 mmol) was dissolved in 8 mL of methanol, sodium borohydride was added therein for a reduction reaction; the reaction material was stirred at room temperature for 3 hours, 5 mL of water was added therein, then the material was adjusted with a concentrated hydrochloric acid to pH 3-4, 10 mL of n-hexane was added therein, the material was stirred at room temperature overnight for a cyclization reaction; an n-hexane layer was separated, washed with water to neutral, dried and concentrated, and purified (petroleum ether: ethyl acetate=50:1) by column chromatography, to obtain a compound 2 (300 mg, and yield: 60.1%).[α]_{D}²⁰ = 29.3 ° (c 1.32, CHCl3), 1H NMR (600 MHz, CDCl₃) δ 0.90 (s, 3 H), 1.04-1.09 (m, 1 H), 1.21-1.25 (m, 5 H), 1.36 (s, 3 H), 1.62-1.76 (m, 7 H), 1.94-2.18 (m, 7H), 5.60-5.61 (m, 1 H); ¹³C NMR (150 MHz, CDCl₃) δ 135.1, 127.2, 85.1, 81.0, 44.10, 37.0, 34.4, 33.8, 33.6, 32.9, 32.6, 30.4, 30.1, 24.4, 22.9, 22.5, 21.9, 21.3, 21.2. HR-MS *m*/*z* (ESI) 299.1990 [M+H-H2O]⁺, C₁₈H₂₆F₃ Calcd. for 299.1981.

### Example 2:

### Step 1

### Preparation of (6R,9R)6-methyl-9-(1-hydroxyisopropyl)-2-(4-fluorobutyl)dicyclo[4.4.0]deca-1-ene-3-one (3)

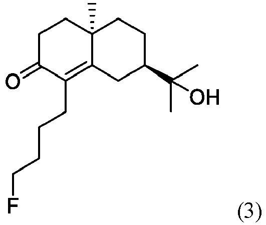

Under argon protection, a compound (500 mg, and 2.25 mmol) shown in Formula (I-a) and KOH (171 mg, and 3.0 mmol) were dissolved in tert-butanol, and heated to reflux, 5 mL of 4-bromo-1-fluorobutane (426 mg, and 2.75 mmol) solution was dropwise added therein, and it was continued to react for 1 h after dropping. The reaction material was cooled, 10 mL of distilled water was added therein, then the material was neutralized with 1N hydrochloric acid until pH=7, and evaporated to remove the solvent, the residue after evaporation was dissolved in ether, an ether layer was washed with saturated sodium chloride, dried with anhydrous sodium sulfate, and evaporated to remove the solvent. A crude product was purified by silica gel column chromatography, and eluted with a mixed solvent of petroleum ether/ethyl acetate (10:1), to obtain a required compound 3 (421 mg, and yield: 63%).[α]_{D}²⁰ = -28.7° (c 0.195, CH₃OH), ¹H NMR (400 MHz, CDCl3) δ 1.17 (s, 3 H), 1.21 (s, 3 H), 1.23 (s, 3 H), 1.32-1.50 (m, 4 H), 1.63-1.69 (m, 3 H), 1.71-1.79 (m, 2 H), 1.85-1.93 (m, 2 H), 2.23-2.30 (m, 1 H), 2.35-2.46 (m, 3 H), 2.52-2.61 (m, 1 H), 2.63-2.69 (m, 1 H), 4.34-4.38 (m, 1 H), 4.46-4.50 (m, 1 H). ¹³C NMR (100 MHz, CDCl₃) δ 198.3, 166.6, 134.2, 85.0, 83.3, 73.6, 44.1, 37.0, 36.6, 35.8, 34.3, 30.6, 30.3, 29.0, 27.2, 27.0, 26.0, 25.0, 24.9, 24.8, 21.3. HR-MS *m*/*z* (ESI) 297.2232 [M+H]⁺, C₁₈H₃₀O₂F Calcd. for 297.2224.

### Step 2

### Preparation of (1R,6S,9R)6,10,10-trimethyl-2-(4-fluorobutyl)-11-oxatricyclo[7.2.1.0^{1,6}]dodec-2-ene (4)

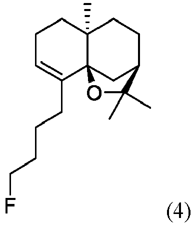

The compound 3 (400 mg, and 1.35 mmol) was dissolved in 5 mL of methanol, sodium borohydride (205 mg, and 5.4 mmol) was added therein for a reduction reaction; the reaction material was stirred at room temperature for 3 hours, 2 mL of water was added therein, then the material was adjusted with a concentrated hydrochloric acid to pH 3-4, 7 mL of n-hexane was added therein, the material was stirred at room temperature overnight for a cyclization reaction; an n-hexane layer was separated, washed with water to neutral, dried and concentrated, and purified by column chromatography (petroleum ether: ethyl acetate=50:1), to obtain a compound 4 (227 mg, and yield: 60.0%). [α]_{D}²⁰ = 20.0° (c 0.5, CHCl3), ¹H NMR (600 MHz, CDCl₃) δ 0.89 (s, 3 H), 1.02-1.07 (m, 1 H), 1.16-1.19 (m, 1 H), 1.22 (s, 3 H), 1.36 (s, 3 H), 1.41-1.68 (m, 2 H) 1.61-1.77 (m, 7 H), 1.93-2.03 (m, 5 H), 2.16-2.20 (m, 1 H), 4.36-4.39 (m, 1 H), 4.48-4.60 (m, 1 H), 5.60-5.61 (m, 1 H). ¹³C NMR (150 MHz, CDCl₃) δ 136.0, 126.7, 85.3, 85.0, 83.4, 80.9, 44.2, 37.0, 34.6, 32.9, 32.7, 30.8, 30.7, 30.5, 24.7, 24.5, 23.0, 22.7, 22.0. HR-MS *m*/*z* (ESI) 263.2167 [M+H-H2O]⁺, C₁₈H₂₈F Calcd. for 263.2170.

### Example 3:

### Step 1

### Preparation of (6R,9R)6-methyl-9-(1-hydroxyisopropyl)-2-(5,5,5-trifluoropentyl)dicyclo[4.4.0]deca-1-ene-3-one (5)

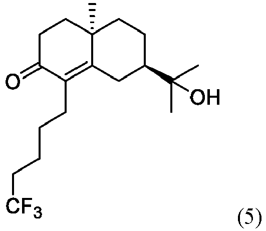

Under argon protection, a compound (1000 mg, and 4.5 mmol) shown in Formula (I-a) and potassium tert-butoxide (758 mg, and 6.75 mmol) were dissolved in 10 mL of tert-butanol, and heated to reflux, 10 mL of a tert-butanol solution of 5-bromo-1,1,1-trifluoropentane (1.108 g, and 5.40 mmol) was dropwise added therein, and it was continued to react for 1 h after dropping. The reaction material was cooled, 10 mL of distilled water was added therein, then the material was neutralized with 1N hydrochloric acid until pH=7, and evaporated to remove the solvent, the residue after evaporation was dissolved in ether, an ether layer was washed with saturated sodium chloride, dried with anhydrous sodium sulfate, and evaporated to remove the solvent. A crude product was purified by silica gel column chromatography, and eluted with a mixed solvent of petroleum ether/ethyl acetate (10:1), to obtain a product 5 (950 mg, and yield: 61%).[α]_{D}²⁰ = -63.3 ^{o} (c 2.4, CHCl3), ¹H NMR (600 MHz, CDCl3) δ 1.16 (s, 3 H), 1.21 (s, 3 H), 1.23 (s, 3 H), 1.24-1.28 (m, 1 H), 1.30-1.45 (m, 4 H), 1.48-1.66 (m, 6 H), 1.72-1.77 (m, 1 H), 1.85-1.93 (m, 2 H), 1.99-2.09 (m, 2 H), 2.20-2.27 (m, 1 H), 2.35-2.44 (m, 3 H), 2.52-2.67 (m, 2 H). ¹³C NMR (150 MHz, CDCl₃) δ 198.2, 165.7, 133.8, 73.4, 44.0, 36.9, 36.5, 35.8, 34.2, 29.0, 28.2, 27.1, 26.9, 25.0, 24.9, 21.9, 21.8, 21.2. HR-MS *m*/*z* (ESI) 347.2189 [M+H]⁺, C₁₉H₃₀O₂F₃ Calcd. for 347.2192.

### Step 2

### Preparation of (1R,6S,9R)6,10,10-trimethyl-2-(5,5,5-trifluoropentyl)-11-oxatricyclo[7.2.1.0^{1,6}]dodec-2-ene (6)

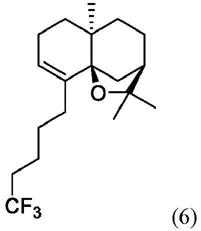

The product 5 (461 mg, and 1.61 mmol) was dissolved in 6 mL of methanol, sodium borohydride was added therein for a reduction reaction; the reaction material was stirred at room temperature for 3 hours, 5 mL of water was added therein, then the material was adjusted with a concentrated hydrochloric acid to pH 3-4, 10 mL of n-hexane was added therein, the material was stirred at room temperature overnight for a cyclization reaction; an n-hexane layer was separated, washed with water to neutral, dried and concentrated, and purified by column chromatography (petroleum ether: ethyl acetate=50:1), to obtain a final product 6 (258 mg, and yield: 55.6%). [α]_{D}²⁰ = 17.0 ° (c 2.1, CHCl₃), ¹H NMR (600 MHz, CDCl3) δ 0.91 (s, 3 H), 1.05-1.09 (m, 1 H), 1.24-1.34 (m, 7 H), 1.37 (s, 3 H), 1.58-1.73 (m, 8 H), 1.95-2.21 (m, 6 H), 5.60 (brs, 1 H); ¹³C NMR (150 MHz, CDCl₃) δ 135.6, 126.5, 85.2, 80.9, 44.1, 37.0, 34.5, 33.8, 33.6, 32.9, 32.6, 30.7, 30.4, 28.1, 24.4, 22.9, 22.5, 22.1, 21.9. HR-MS *m*/*z* (ESI) 313.2134 [M+H-H2O]⁺, C₁₉H₂₈F₃ Calcd. for 313.2138.

### Example 4:

### Step 1

### Preparation of (6R,9R)6-methyl-9-(1-hydroxyisopropyl)-2-(4,4,4-trifluorobutyl)-4,4,10,10-tetradeuteriodicyclo[4 .4.0]deca-1-ene-3-one (7)

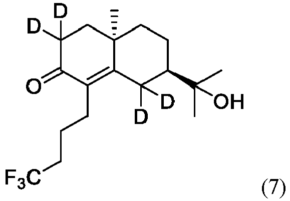

The compound 1 (298 mg, and 0.90 mmol) was dissolved in 3 mL of deuterated methanol, sodium methoxide (49 mg, and 0.90 mmol) was added therein, and under argon protection, the material was stirred overnight at room temperature; the obtained material was adjusted with 1N hydrochloric acid to pH 7, methanol was evaporated, then the obtained material was washed sequentially with saturated sodium bicarbonate solution and saturated sodium chloride, dried with anhydrous sodium sulfate, and evaporated to remove the solvent. A crude product was purified by silica gel column chromatography (petroleum ether: ethyl acetate=20:1) to obtain a yellow oil-like substance 7 (290 mg, and yield: 97%).[α]_{D}²⁰ = -66.6 ° (c 0.89, CHCl₃), ¹H NMR (600 MHz, CDCl3) δ 1.21 (s, 3 H), 1.25 (s, 3 H), 1.28 (s, 3 H), 1.43-1.48 (m, 1 H), 1.50-1.68 (m, 8 H), 1.78-1.83 (m, 1 H), 1.92-1.96 (m, 2 H), 2.06-2.12 (m, 2 H), 2.32-2.37 (m, 1 H), 2.46-2.51 (m, 1 H). ¹³C NMR (150 MHz, CDCl₃) δ 198.2, 166.4, 133.3, 126.3, 73.4, 43.9, 36.9, 36.2, 35.8, 33.7, 33.5, 29.0, 27.0, 25.0, 24.4, 21.5, 21.2. HR-MS *m*/*z* (ESI) 337.2260 [M+H]⁺, C₁₈H₂₄D₄O₂F₃ Calcd. for 337.2287.

### Step 2

### Preparation of (1R,6R,9R)6,10,10-trimethyl-2-(4,4,4-trifluorobutyl)-4,4,10-trideuterium-11-oxatricyclo[7.2.1.0^{1,6} ]dodec-2-ene (8)

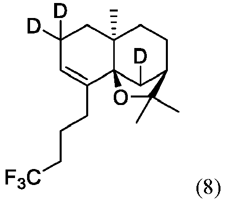

Lithium aluminium hydride (32 mg, and 0.83 mmol) was suspended in 3 mL of anhydrous tetrahydrofuran, and under an ice bath condition, a tetrahydrofuran solution of the yellow oil-like substance 7 (280 mg, and 0.83 mmol) was dropwise added therein, after dropping, the temperature was raised to room temperature and the material was stirred for 3 hours to perform a reduction reaction; 3 mL of water was added therein, the obtained material was adjusted with a concentrated hydrochloric acid to pH 3-4, 3 mL of n-hexane was added therein, then the material was stirred at room temperature overnight for a cyclization reaction; an n-hexane layer was separated, washed with water to neutral, dried and concentrated, and purified by column chromatography (petroleum ether: ethyl acetate=50:1) to obtain a final product 8 (200 mg, yield: 76%). [α]_{D}²⁰ = 29.3 ° (c 1.32, CHCl3), ¹H NMR (600 MHz, CDCl3) δ 0.93 (s, 3 H), 1.08-1.10 (m, 1 H), 1.22-1.25 (m, 4 H), 1.39 (s, 3 H), 1.67-1.75 (m, 6 H), 1.96-1.98 (m, 1 H), 2.05-2.19 (m, 4 H), 5.63 (s, 1H); ¹³C NMR (150 MHz, CDCl₃) δ 135.2, 127.0, 85.0, 81.0, 44.0, 36.9, 34.4, 33.8, 33.6, 32.6, 30.4, 30.1, 24.3, 22.9, 21.9, 21.2, 21.2. HR-MS *m*/*z* (ESI) 302.2161 [M+H-H2O]⁺, C₁₈H₂₃D₃F₃ Calcd. for 302.2169.

### Example 5:

### Preparation of (1R,2R/S,6S,9R)6,10,10-trimethyl-2-(4,4,4-trifluorobutyl)-11-oxatricyclo[7.2.1.0¹⁶]dodecane (9)

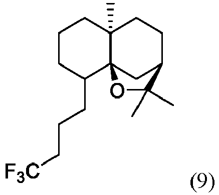

The compound 2 (50 mg, and 0.16 mmol) was dissolved in 5 mL of ether, 30 mg of 10% palladium carbon was added therein, the material was hydrogenated at room temperature and atmospheric pressure for 7 hours, then palladium carbon was filtered, and the material was evaporated to remove the solvent, and purified by column chromatography (petroleum ether: ethyl acetate=50:1) to obtain a final product 9 (15 mg, and yield: 30%).[α]_{D}²⁰ = 11.2 ° (c 0.68, CHCl₃), ¹H NMR (600 MHz, CDCl3) δ 0.86 (s, 3 H), 1.10-1.14 (m, 4 H), 1.20 (m, 3 H), 1.27-1.77 (m, 14 H), 1.81-2.14 (m, 4 H), 2.31-2.66 (m, 1 H); HR-MS *m*/*z* (ESI) 301.2132 [M+H-H2O]⁺, C₁₈H₂₈F₃ Calcd. for 301.2138.

### Example 6:

### Preparation of (1R,6R,9R)6,10,10-trimethyl-2-(4,4,4-trifluorobutyl)-11-oxatricyclo[7.2.1.0^{1,6}]dodec-2-ene-4-one (10)

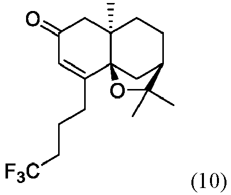

The compound 2 (100 mg, and 0.32 mmol) was dissolved in 5 mL of dichloromethane, 100 mg of pyridinium chlorochromate (PCC) was added therein, the obtained material was stirred overnight at room temperature, filtered with a silica gel pad, evaporated to remove the solvent, and purified by column chromatography (petroleum ether: ethyl acetate=50:1) to obtain a final product 10 (80 mg, and yield: 76%), [α]_{D}²⁰ = 28.6 ° (c 1.01, CHCl₃), ¹H NMR (600 MHz, CDCl3) δ 0.90 (s, 3 H), 1.04-1.09 (m, 1 H), 1.21-1.25 (m, 5 H), 1.36 (s, 3 H), 1.62-1.76 (m, 6 H), 1.94-2.18 (m, 3 H), 2.38-2.45 (m, 3 H), 5.60-5.61 (m, 1 H); MS *m*/*z* (ESI) 313.2 [M+H-H₂O]⁺.

### Example 7:

### Preparation of (1R,4R/S,6R,9R)6,10,10-trimethyl-2-(4,4,4-trifluorobutyl)-4-hydroxy-11-oxatricyclo[7.2.1.0^{1,6}]do dec-2-ene (11)

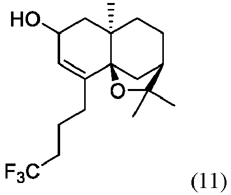

The final product 10 (50 mg, and 0.15 mmol) was dissolved in 2 mL of methanol, sodium borohydride (20 mg, and 0.53 mmol) was added therein, the obtained material was stirred at room temperature for 2 hours, then 3 mL of distilled water was added, the material was adjusted with 2M hydrochloric acid to pH 3-4, dichloromethane was added for extraction, the material was washed sequentially with water and saturated sodium chloride, dried with anhydrous sodium sulfate, filtered, evaporated to remove the solvent, and purified by column chromatography (petroleum ether: ethyl acetate=20:1) to obtain a final product 11 (30 mg, and yield: 60%), [α]_{D}²⁰ = 20.7 ° (c 1.03, CHCl₃), ¹H NMR (600 MHz, CDCl₃) δ 0.90 (s, 3 H), 1.04-1.09 (m, 1 H), 1.21-1.25 (m, 5 H), 1.36 (s, 3 H), 1.51-1.80 (m, 7 H), 1.94-2.18 (m, 2 H), 2.38-2.45 (m, 3 H), 4.05-4.08 (m, 1 H), 5.60-5.61 (m, 1 H); MS *m*/*z* (ESI) 298.2 [M+2H-2H₂O]⁺.

### Example 8: Biological evaluations

### I. Pharmacodynamic experiment

The novel agarofuran derivative compound shown in Formula (I) of the present application shows excellent effects in animal antianxiety models, such as an elevated plus maze test for rats. The active ingredient used in the following pharmacological experiment, the compound shown in Formula (I) of the present application, is the final product in Example 1 of the present application, namely (1R,6S,9R)6,10,10-trimethyl-2-(4,4,4-trifluorobutyl)-11-oxatricyclo[7.2.1.0^{1,6}]dodec-2-ene (hereinafter referred to as Compound 2). Compound 2 and an excipient PVP (povidone K30) were mixed in an ethanol solution in a mass ratio of 1:15, spin-dried, and prepared into a preparation form of Compound 2 (the following Compound 2 refers to the preparation form of Compound 2). The preparation form of which the active ingredient was 4-butyl-α-agarofuran (BAF for short) was used as a positive drug, a preparation method for this preparation was the same as that of the preparation of Compound 2, and the following BAF refers to this preparation of BAF. PVP was a blank control. In addition, the administration dose was calculated according to a crude drug.

### Elevated plus maze (EPM) test

This model is one of the most commonly used animal models in the research of antianxiety drugs. The elevated plus maze is composed of an open arm (30×5×0.5 cm) and an closed arm (30×8×15 cm) that are crossed in a positive cross shape, and the instrument is suspended 50 cm above the ground. Due to the narrowness, suspension, and brightness of the open arm, there may be internal "fear" or conflict in the open arm, and on the contrary, it more complies with the nature of rodents in the closed arm. 5 min after administration, a mouse is placed in the center of the maze, and its head faces to the open arm. Open-arm and closed-arm entries and residence times in both arms during the experimental period (usually 5 min) are recorded respectively. The percentages of the open arm entries and the open arm residence times respectively accounting for the total entries (sum of the open arm and closed arm entries) and the total time (sum of the open arm and closed arm residence times) are calculated, as an indicator for evaluating the anxiety. Generally speaking, a drug is considered to have antianxiety effects in the case that the open arm cumulative residence time is prolonged without reducing the open arm and closed arm entries.

The experiment used male Institute of Cancer Research (ICR) mice (purchased from Beijing Weitong Lihua Experimental Animal Technology Co., Ltd.), the weight was 18-20 g, the mice were randomly divided into 7 groups according to the weight, and there were 13 mice in each group. Grip adaptation training was conducted on the mice before the experiment. Before the experiment, the mice were fasted for 12 hours. It was administered by an intraperitoneal injection mode. 4-butyl-α-agarofuran (BAF for short) was used as a positive drug, and PVP was a blank control. The specific grouping and disposal modes are as follows:

**Table 1.Animal grouping and disposal**

| Group | Number of animals | Disposal |
|---|---|---|
| Control | 13 | a PVP excipient, administered 5 min before entering the maze |
| BAF-L | 13 | 2 mg/kg, administered 5 min before entering the maze |
| BAF-M | 13 | 4 mg/kg, administered 5 min before entering the maze |
| BAF-H | 13 | 8 mg/kg, administered 5 min before entering the maze |
| Compound 2-L | 13 | 2.4 mg/kg, administered 5 min before entering the maze |
| Compound 2-M | 13 | 4.8 mg/kg, administered 5 min before entering the maze |
| Compound 2-H | 13 | 9.6 mg/kg, administered 5 min before entering the maze |

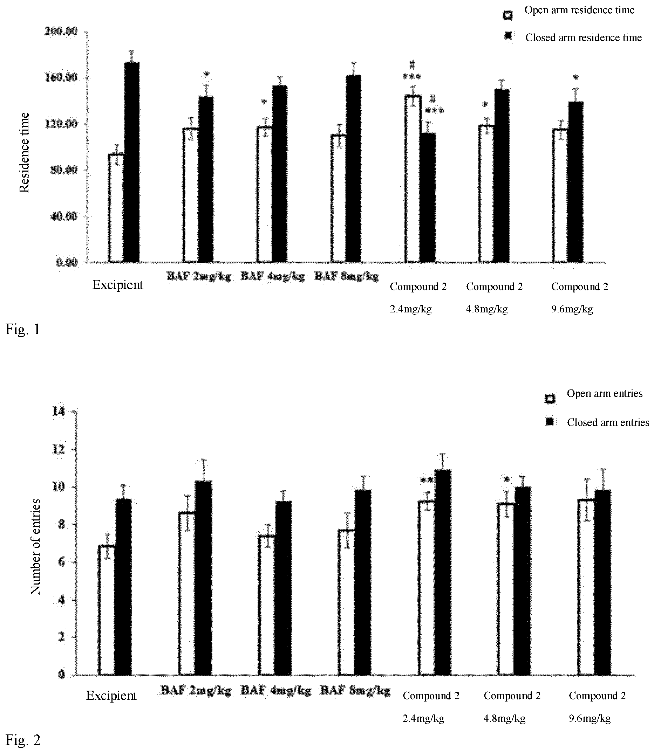

During detection, after intraperitoneal injection of BAF/Compound 2, each mouse was immediately placed in an open animal box and allowed to move freely for 5 min, then the mouse was placed on a central platform of the elevated plus maze, its head faced to a fixed open arm. The detection was started, timed for 5 min, and recorded with a supermaze software by using a camera head. After all the experiments were completed, the residence times (s) of the mice in the open arm and the closed arm, the open arm and closed arm entries, and the time ratios and frequency ratios of the open arm and closed arm entries were recorded with the supermaze software. 1 was added to the number of entry arms with a latency of 0. The experimental results are shown in Table 2 and Figs. 1-3:

**Table 2. Dose-effect relationship of BAF and Compound 2 administered intraperitoneally on elevated plus maze**

| **Group** | **Open arm** | | **Closed arm** | | **Open arm/closed arm** | |
|---|---|---|---|---|---|---|
| | **Residence time (sec)** | **Number of entries** | **Residence time (sec)** | **Number of entries** | **Time ratio** | **Frequenc y ratio** |
| Excipient | 93.42±8.81 | 6.85±0.62 | 173.26±10.32 | 9.38±0.6 8 | 0.60±0.0 9 | 0.77±0.09 |
| BAF-L(2mg/kg ) | 115.97±9.71 | 8.62±0.92 | 143.9±9.32^{∗} | 10.31±1. 13 | 0.93±0.1 6 | 0.85±0.06 |
| BAF-M(4mg/k g) | 117.29±7.44 ^{∗} | 7.38±0.58 | 153.35±7.35 | 9.23±0.5 7 | 0.82±0.1 1 | 0.82±0.07 |
| BAF-H(8mg/kg ) | 110.07±9.77 | 7.69±0.91 | 162.19±10.92 | 9.85±0.7 2 | 0.76±0.1 0 | 0.76±0.05 |
| Compound 2-L (2.4 mg/kg) | 144.06±8.1 0^{∗∗∗#} | 9.23±0.47^{∗} * | 112.68±8.68^{∗ ∗∗#} | 10.92±0. 84 | 1.48±0.2 4^{∗∗} | 0.89±0.06 |
| Compound 2-M (4.8 mg/kg) | 118.65±6.32 ^{∗} | 9.08±0.69^{∗} | 149.91±8.22 | 10.00±0. 57 | 0.86±0.1 1^{∗} | 0.93±0.07 |
| Compound 2-H (9.6 mg/kg) | 115.0±8.07 | 9.31±1.12 | 139.13±11.37 ^{∗} | 9.85±1.0 8 | 0.93±0.1 2^{∗} | 0.99±0.10 |

| | | | | | | |
|---|---|---|---|---|---|---|
| *P <0.05, **P <0.01, ***P < 0.001 compared with the excipient group; and ^{#}P < 0.05 compared with the BAF (2 mg/kg) group. | | | | | | |

The experimental results show that compared with BAF, Compound 2 to be tested has significant antianxiety effects when injected intraperitoneally at doses of 2.4, 4.8, and 9.6 mg/kg (equimolar doses of 2, 4, and 8 mg/kg of BAF). Compound 2 has better efficacy than BAF at both low and medium doses, especially at a low dose, and there are statistical differences between the efficacy results.

In addition, the antianxiety activities of the compound 6 and the compound 8 were respectively evaluated according to the above scheme. As shown in Tables 3 and 4, compared with BAF, the open-arm residence times and entries are both prolonged, and there are also statistical differences at a low dose.

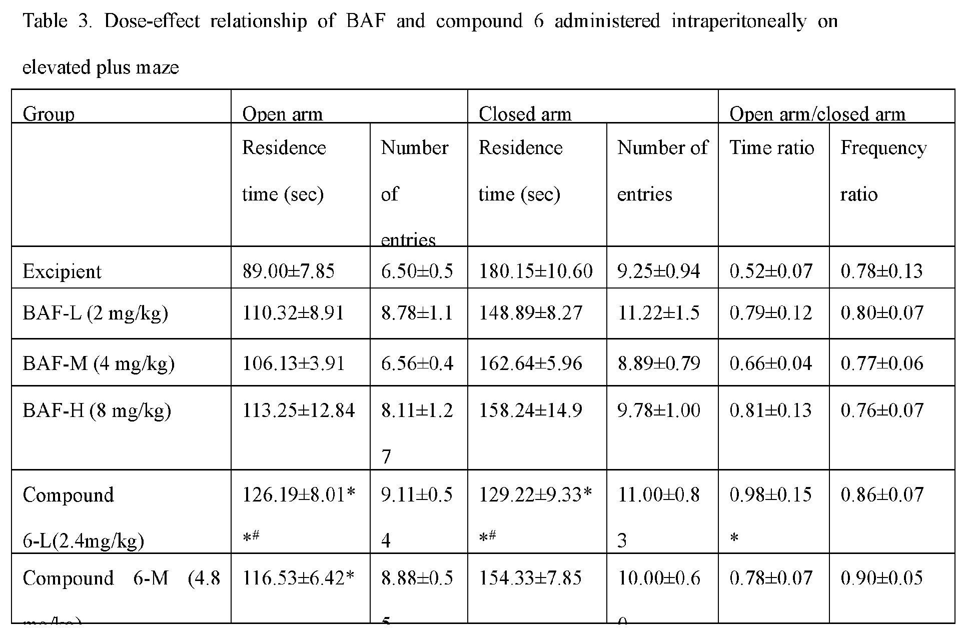

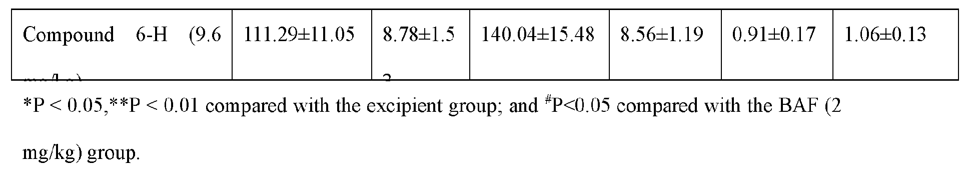

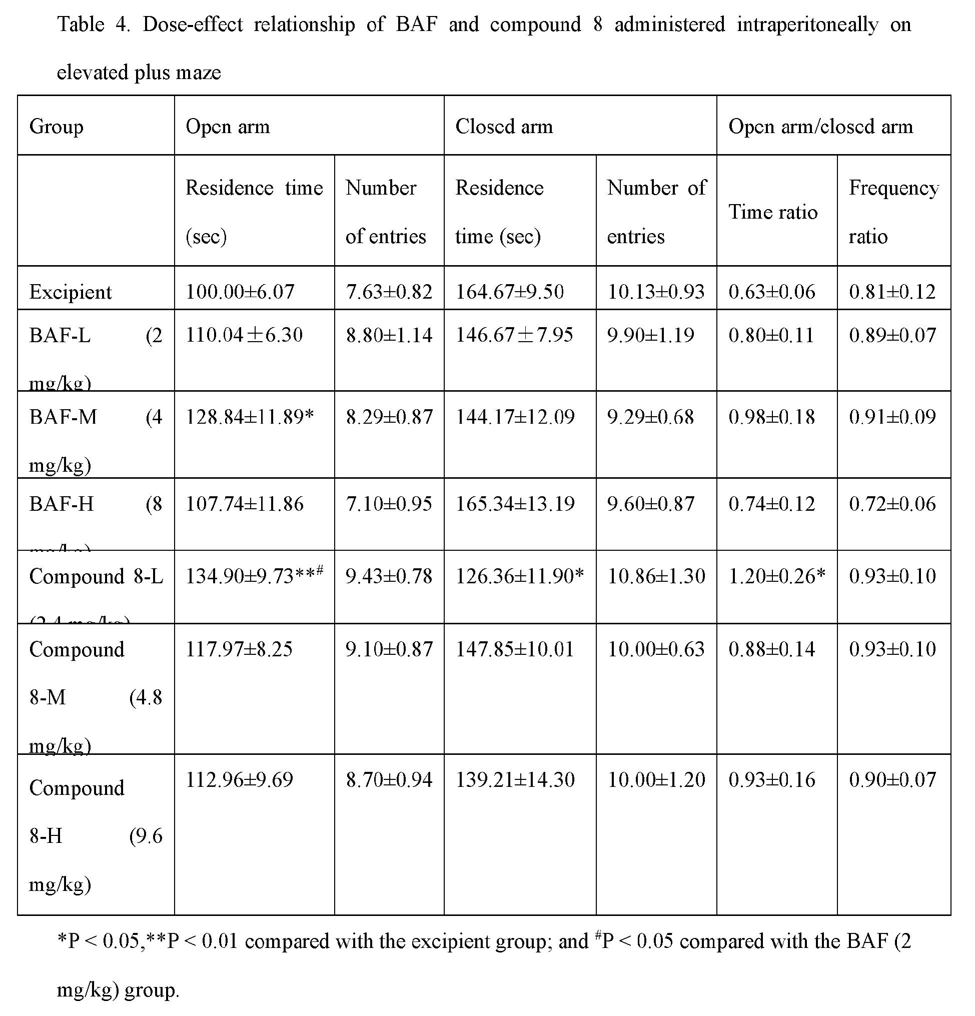

### II. Pharmacokinetic experiment

Research on plasma pharmacokinetics of compound 2 after intraperitoneal injection in mouse

### 1. Experimental material

Positive control drug 4-butyl-α-agarofuran preparation (BAF) and Compound 2 preparation were administered intraperitoneally at a dose of 10mg/kg/10ml (calculated according to the crude drug), and a suspension was prepared by ultrasonic grinding with double distilled water.

Male ICR mice, with a weight of 21-24 g, were purchased from SPF (Beijing) Biotechnology Co., Ltd.

### 2. Experimental method

### 1) Sample collection

12 mice were randomly divided into 4 groups, there were 3 parallel cross blood sampling points in each group (5 points in groups 1 and 3, and 6 points in groups 2 and 4), it was administered according to the weight, and blood was collected from the orbital venous plexus at 5 min, 10 min, 15 min, 30 min, 1 h, 2 h, 4 h, 6 h, 8 h, 12 h, and 24 h after administration. The bloods at each time point were collected into Eppendorf (EP) tubes anticoagulated with heparin, and placed on ice and centrifuged at 8000 r/min×5 min, to separate plasma for later use.

### 2) Standard curve preparation

20 µl of mouse blank plasma was taken, 55 µl of acetonitrile containing an internal standard solution (200 ng/mL, BAF crude drug and Compound 2 crude drug are mutual internal standards, namely BAF was used as the internal standard of Compound 2, and Compound 2 was used as the internal standard of BAF, similarly hereinafter) is added, 5 µl of a methanol working solution of the compound BAF or Compound 2 with different concentrations was respectively added and mixed uniformly, so that its final plasma concentrations were 0.2, 0.5, 2, 5, 10, 25, 50, 100, 200, 500, 750, and 1000 ng/ml respectively, centrifugation was conducted twice at a high speed of 13000 r/min×5 min, and 5 µl of a supernatant was taken for UPLC-MS/MS analysis.

### 3) Sample treatment

10 µL of plasma was taken, 30 µL of acetonitrile containing internal standard solution (100 ng/mL) was added to precipitate proteins, after the material was centrifuged twice at a high speed of 13000 r/min×5 min, 5 µl of a supernatant was taken for UPLC-MS/MS analysis.

### 4) UPLC-MS/MS determination

Chromatographic column: Symmetry C8 (2.1 mm×50 mm, 3.5 µm), column temperature: 25 °C, mobile phase: acetonitrile (0.1% formic acid): water (0.1% formic acid) = 70:30 gradient elution, flow rate: 0.2 mL/min, Parallel Reaction Monitoring (PRM) detection, BAF: m/z 263.2 → 245.2; and Compound 2: m/z 317.2→299.2.

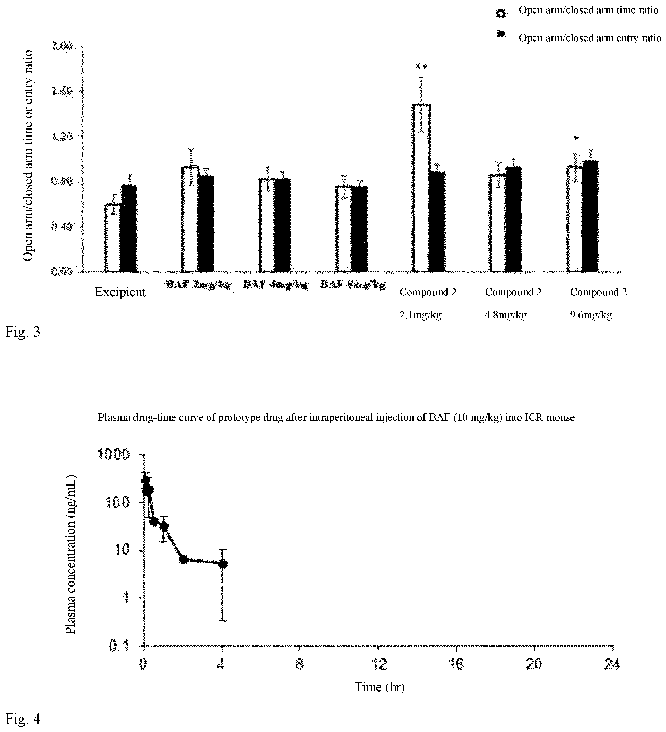

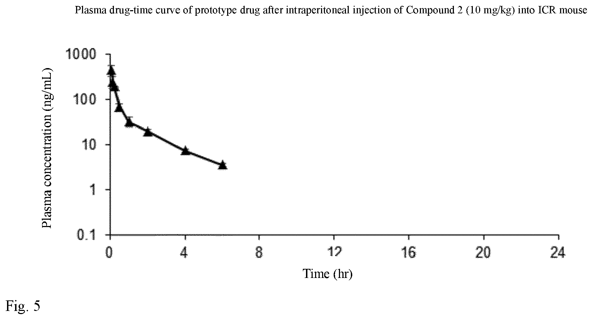

The experimental results are shown in Tables 5-8 and Figs. 4-5. After intraperitoneal injection of BAF or Compound 2 (10 mg/kg) in mouse, the absorption is faster, and reaches a peak within 5-15 min, Cₘₐₓ and AUC₍₀₋ₜ₎ of Compound 2 are 150% and 142% of BAF respectively, the exposure dose is increased, and the mean residence time (MRT)₍₀₋ₜ₎ is prolonged from 1.05 to 1.32 hours, while T_{1/2} is prolonged from 1.29 to 1.61 hours. Therefore, it is believed that Compound 2 has better pharmacokinetic properties than BAF.

In addition, the pharmacokinetic properties of Compounds 6 and 8 are also evaluated according to the above method. Cₘₐₓ of Compound 6 is 342 ng/mL, AUC₍₀₋ₜ₎ is 142 h*ng/mL, and T_{1/2} is 1.39 hours. Cₘₐₓ of Compound 8 is 350 ng/mL, AUC₍₀₋ₜ₎ is 148 h*ng/mL, and T_{1/2} is 1.41 hours, it is indicated that the pharmacological properties of Compounds 6 and 8 are also superior to BAF.

**Table 5. Chronological changes of prototype drug plasma after intraperitoneal injection of BAF (10 mg/kg) in mouse**

| **Time (h)** | **Concentration (ng/mL)** | | | **Mean (ng/mL)** | **SD (ng/mL)** | **CV (%)** |
|---|---|---|---|---|---|---|
| | **1** | **2** | **3** | | | |
| 0.0833 | 413 | 295 | 194 | 301 | 110 | 36.5 |
| 0.167 | 201 | 133 | 204 | 179 | 40 | 22.4 |
| 0.25 | 364 | 132 | 91.8 | 196 | 147 | 75.0 |
| 0.5 | 41.9 | 45.2 | 38.9 | 42.0 | 3.2 | 7.50 |
| 1 | 54.6 | 22.2 | 24.4 | 33.7 | 18.1 | 53.7 |
| 2 | 6.10 | 6.91 | 6.54 | 6.52 | 0.41 | 6.22 |
| 4 | 11.3 | 2.09 | 2.90 | 5.4 | 5.1 | 93.9 |
| 6 | BLOQ | BLOQ | BLOQ | NA | NA | NA |
| 8 | 4.03 | BLOQ | BLOQ | NA | NA | NA |
| 12 | BLOQ | BLOQ | BLOQ | NA | NA | NA |
| 24 | BLOQ | BLOQ | BLOQ | NA | NA | NA |

**Table 6. Pharmacokinetic parameters of prototype drug plasma after intraperitoneal injection of BAF (10 mg/kg) in mouse**

| **PK parameters** | **Unit** | **IP** |
|---|---|---|
| T_{1/2} | h | 1.29 |
| Tₘₐₓ | h | 0.0833 |
| Cₘₐₓ | ng/mL | 301 |
| AUCₗₐₛₜ | h*ng/mL | 129 |
| AUC_{Inf} | h*ng/mL | 139 |
| AUC_{_%Extrap_}obs | % | 7.24 |
| MRT_{Inf_}obs | h | 1.05 |
| AUCₗₐₛₜ/D | h^{∗}mg/mL | 12.9 |

**Table 7. Chronological changes of prototype drug plasma after intraperitoneal injection of Compound 2 (10 mg/kg) in mouse**

| **Time (h)** | **Concentration (ng/mL)** | | | **Mean (ng/mL)** | **SD (ng/mL)** | **CV (%)** |
|---|---|---|---|---|---|---|
| | **1** | **2** | **3** | | | |
| 0.0833 | 359 | 407 | 585 | 450 | 119 | 26.4 |
| 0.167 | 341 | 198 | 214 | 251 | 78 | 31.2 |
| 0.25 | 188 | 201 | 196 | 195 | 7 | 3.36 |
| 0.5 | 81.1 | 59.8 | 67.5 | 69.5 | 10.8 | 15.5 |
| 1 | 24.3 | 41.2 | 32.1 | 32.5 | 8.5 | 26.0 |
| 2 | 19.7 | 17.9 | 22.3 | 20.0 | 2.2 | 11.1 |
| 4 | 7.77 | 8.03 | 6.56 | 7.45 | 0.78 | 10.5 |
| 6 | 3.69 | 3.20 | 3.79 | 3.56 | 0.32 | 8.87 |
| 8 | BLOQ | 3.17 | BLOQ | NA | NA | NA |
| 12 | BLOQ | BLOQ | BLOQ | NA | NA | NA |
| 24 | BLOQ | BLOQ | BLOQ | NA | NA | NA |

**Table 8. Pharmacokinetic parameters of prototype drug plasma after intraperitoneal injection of Compound 2 (10 mg/kg) in mouse**

| **PK parameters** | **Unit** | **IP** |
|---|---|---|
| T_{1/2} | h | 1.61 |
| Tₘₐₓ | h | 0.0833 |
| Cₘₐₓ | ng/mL | 450 |
| AUCₗₐₛₜ | h*ng/mL | 190 |
| AUC_{Inf} | h*ng/mL | 198 |
| AUC_{_%Extrap_}obs | % | 4.16 |
| MRT_{Inf_}obs | h | 1.32 |
| AUCₗₐₛₜ/^{D} | h*mg/mL | 19.0 |

Conclusion: Compound 2, Compound 6, and Compound 8 of the present application all have the antianxiety activity with much higher in vivo exposure dose and half-life compared to BAF, indicating better pharmacokinetic properties. At the same time, at low doses, they all show better efficacy, indicating that the compounds have lower effective dose, and the clinical administration dose may be reduced.

In addition, in the compound shown in General formula (I), R₂, R₃ and R₄ are the metabolic sites of the compound. When it is metabolized in vivo, the C₂-C₉ linear hydrocarbylene, branched hydrocarbylene or cycloalkylene in the position R₂ is easy to be substituted by carbonyl or hydroxyl, and R₃ and R₄ are also easy to be metabolized into carbonyl or hydroxyl. When the C₂-C₉ linear hydrocarbylene, branched hydrocarbylene or cycloalkylene in the position R₂ is unsubstituted by carbonyl or hydroxyl, and the prototype drug of which R₃ and R₄ are hydrogen atoms or deuterium atoms is proved to have activity or better activity in vivo, those skilled in the art may predict that its metabolic form substituted by carbonyl or hydroxyl also has activity or better activity in vivo.

### Industrial Applicability

The present application provides an agarofuran-based compound, a preparation method therefor, and an application thereof, herein by structural modifications of main metabolic sites such as alkyl side chains of 4-butyl-α-agarofuran (BAF), a novel agarofuran-based compound with higher activity, better efficacy, and better pharmacokinetic properties for treatment of mental diseases such as an anxiety disorder is provided.

## Claims

1. An agarofuran-based compound, having a structural formula (I): 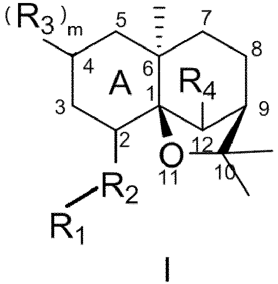
wherein there is no double bond in the ring A or there is one double bond located in the position 2-3 of the ring A;
R₁ is methyl that is monosubstituted, disubstituted, or trisubstituted by fluorine atom(s);
R₂ is C₂-C₉ linear hydrocarbylene, branched hydrocarbylene or cycloalkylene that is unsubstituted, hydroxyl-substituted or carbonyl-substituted, and the linear hydrocarbylene or branched hydrocarbylene is alkylene or alkenylene containing 1-3 double bonds;
m represents the number of R₃ groups linked to carbon in the position 4 of the ring A, m=1 or 2, and when m=1, R₃ is selected from hydrogen atom, carbonyl, or hydroxyl; when m=2, R₃ is deuterium atom; and
R₄ is selected from hydrogen atom, deuterium atom, carbonyl or hydroxyl.

2. The agarofuran-based compound according to claim 1, wherein R₂ is C₂-C₅ linear alkylene that is unsubstituted, hydroxyl-substituted, or carbonyl-substituted; optionally, R₂ is C₃-C₄ linear alkylene that is unsubstituted, hydroxyl-substituted, or carbonyl-substituted; and further optionally, R₂ is C₃ linear alkylene that is unsubstituted, hydroxyl-substituted, or carbonyl-substituted.

3. The agarofuran-based compound according to claim 1, wherein it is selected from the following compounds:

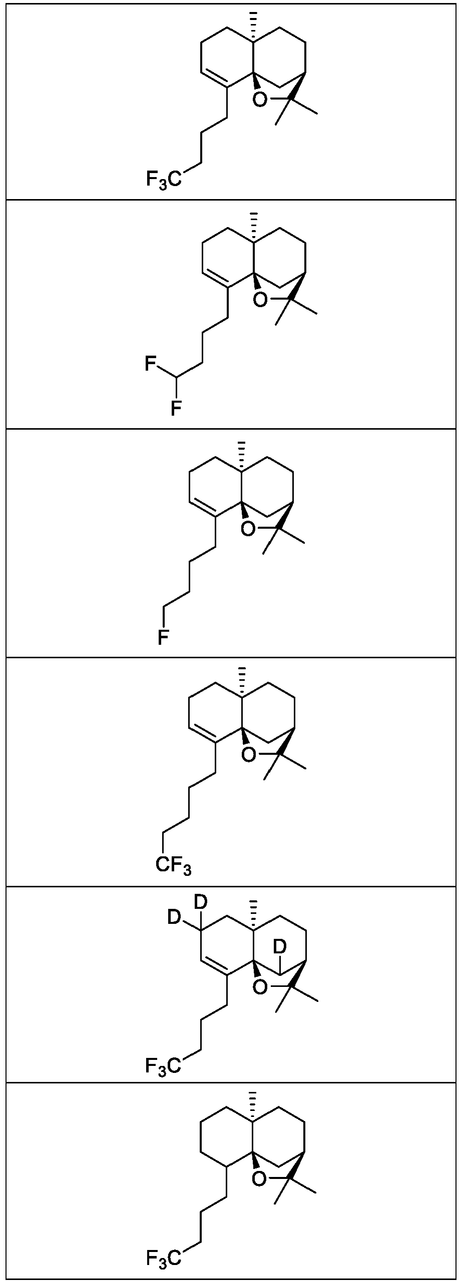

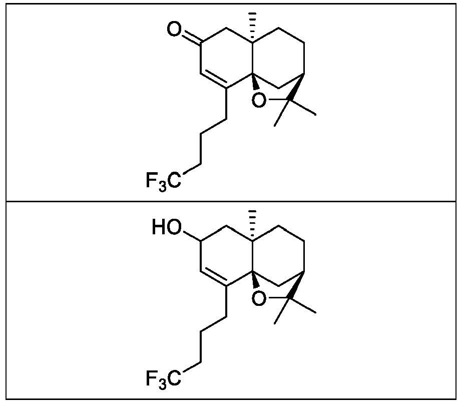

4. A pharmaceutical composition, comprising the agarofuran-based compound according to any one of claims 1-3, a pharmaceutically acceptable salt thereof, or a prodrug thereof, and further comprising at least one of a pharmaceutically acceptable carrier, a diluent, or an excipient.

5. A use of the agarofuran-based compound according to any one of claims 1-3, the pharmaceutically acceptable salt thereof, the prodrug thereof, or the pharmaceutical composition comprising the same in preparation of a drug for preventing and/or treating an anxiety disorder.

6. A preparation method for the agarofuran-based compound according to claim 1, wherein the preparation method is selected from any one of the followings:
(1) when there is one double bond located in the position 2-3 of the ring A of the agarofuran-based compound shown in General formula (I), m=1, and R₃ and R₄ are both hydrogen atoms, the preparation method comprises the following steps: 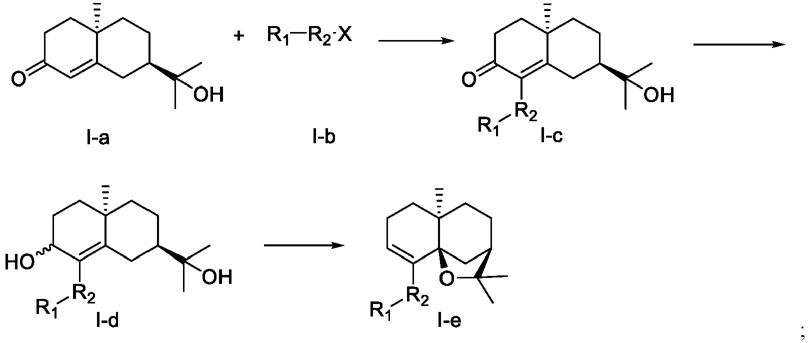
wherein, compounds shown in Formulas (I-a) and (I-b) are subjected to a substitution reaction under an alkaline condition, to obtain a compound shown in Formula (I-c); R₁ is methyl that is monosubstituted, disubstituted, or trisubstituted by fluorine atom(s); R₂ is C₂-C₉ linear hydrocarbylene, branched hydrocarbylene or cycloalkylene that is unsubstituted, hydroxyl-substituted or carbonyl-substituted, and the linear hydrocarbylene or branched hydrocarbylene is alkylene or alkenylene containing 1-3 double bonds; X is halogen, preferably bromine;
the compound shown in Formula (I-c) is subjected to a reduction reaction in the presence of a reducing agent, to obtain a compound shown in Formula (I-d); and
the compound shown in Formula (I-d) is subjected to a cyclization reaction under an acidic condition, to obtain a compound in Formula (I-e);
(2) when there is no double bond in the ring A of the agarofuran-based compound shown in General formula (I), m=1, and R₃ and R₄ are both hydrogen, the preparation method comprises the following steps: the compound (I-e) in the preparation method (1) is subjected to catalytic hydrogenation to obtain a compound shown in Formula (I-f); 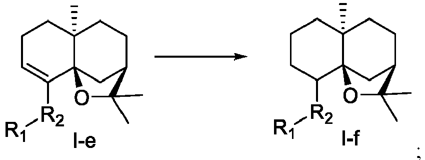
(3) when there is one double bond located in the position 2-3 of the ring A of the agarofuran-based compound shown in General formula (I), m=2, and R₃ and R₄ are both deuterium, the preparation method comprises the following steps: 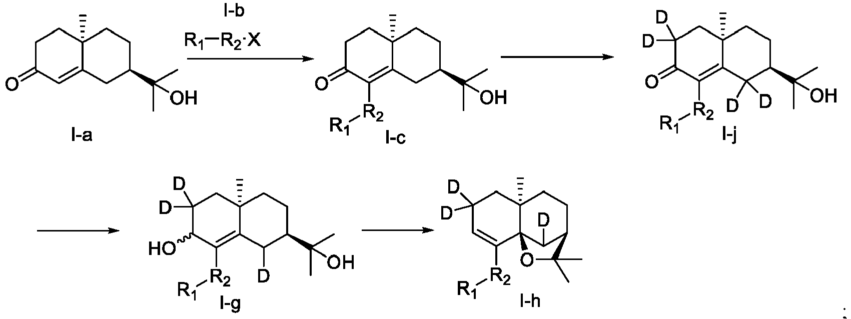
wherein, compounds shown in Formulas (I-a) and (I-b) are subjected to a substitution reaction under an alkaline condition, to obtain a compound shown in Formula (I-c); R₁ is methyl that is monosubstituted, disubstituted, or trisubstituted by fluorine atom(s); R₂ is C₂-C₉ linear hydrocarbylene, branched hydrocarbylene or cycloalkylene that is unsubstituted, hydroxyl-substitutedor carbonyl-substituted, and the linear hydrocarbylene or branched hydrocarbylene is alkylene or alkenylene containing 1-3 double bonds; X is halogen, preferably bromine;
the compound shown in Formula (I-c) is subjected to a deuteration reaction with deuterated alcohol under an alkaline condition, to generate a compound shown in Formula (I-j);
the compound shown in Formula (I-j) is subjected to a reduction reaction in the presence of a reducing agent, to obtain a compound shown in Formula (I-g); and
the compound shown in Formula (I-g) is subjected to a cyclization reaction under an acidic condition, to obtain a compound shown in Formula (I-h);
(4) when there is no double bond in the ring A of the agarofuran-based compound shown in General formula (I), m=2, and R₃ and R₄ are both deuterium, the preparation method comprises the following steps: the compound (I-h) in the preparation method (3) is subjected to catalytic hydrogenation to obtain a compound shown in Formula (I-i); 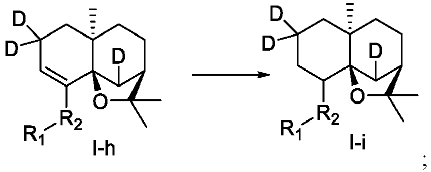
(5) when there is one double bond in the ring A of the agarofuran-based compound shown in General formula (I), m=1, R₃ is carbonyl, and R₄ is hydrogen, the preparation method comprises the following steps: the compound shown in Formula (I-e) in the preparation method (1) is subjected to an oxidation reaction in the presence of an oxidant, to obtain a compound shown in Formula (I-k); 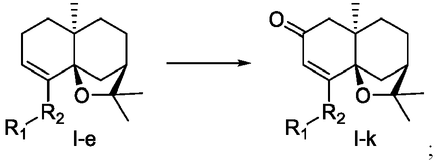
(6) when there is one double bond in the ring A of the agarofuran-based compound shown in General formula (I), m=1, R₃ is hydroxyl, and R₄ is hydrogen, the preparation method comprises the following steps: the compound shown in Formula (I-k) in the preparation method (5) is subjected to a reduction reaction in the presence of a reducing agent, to obtain a compound shown in Formula (I-m);

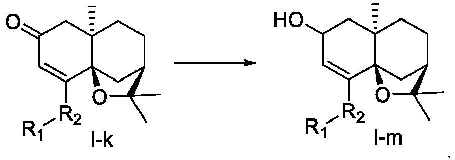

7. The preparation method according to claim 6, wherein R₂ is C₂-C₅ linear alkylene that is unsubstituted, hydroxyl-substituted, or carbonyl-substituted; optionally, R₂ is C₃-C₄ linear alkylene that is unsubstituted, hydroxyl-substituted, or carbonyl-substituted; and further optionally, R₂ is C₃ linear alkylene that is unsubstituted, hydroxyl-substituted, or carbonyl-substituted.

8. The preparation method according to claim 6, wherein R₁ and R₂ are selected from any one of the following combinations:
1) R₁ = -CF₃, R₂ = -CH₂CH₂CH₂-
2) R₁ = -CH₂F, R₂ = -CH₂CH₂CH₂-
3) R₁ = -CHF₂, R₂ = -CH₂CH₂CH₂-
4) R₁ = -CF₃, R₂ = -CH₂CH₂CH₂CH₂-

9. The preparation method according to claim 6, wherein the reagent for providing an alkaline condition comprises an inorganic base, and the inorganic base comprises: one or more of sodium hydride, sodium hydroxide, potassium hydroxide, sodium tert-butanol, and potassium tert-butanol; and/or, the reducing agent comprises: one or more of sodium borohydride, potassium borohydride, and lithium aluminum hydride;
and/or, the reagent for providing an acidic condition comprises: one or more of trifluoroacetic acid, hydrochloric acid, sulfuric acid, phosphoric acid and p-toluenesulfonic acid;
and/or, the metal catalyst for the catalytic hydrogenation comprises: one or more of palladium carbon, rhodium carbon, platinum dioxide, and Raney nickel;
and/or, the oxidant for the oxidation reaction comprises: one or more of oxygen, hydrogen peroxide, peroxide, and Sarrett reagent.

10. The preparation method according to claim 6, wherein the solvents used in the preparation method (1) comprise: Step 1: one or more of methanol, ethanol, isopropanol, and tert-butanol;
Step 2: one or more of ether and tetrahydrofuran; and Step 3: one or more of n-hexane and petroleum ether;
and/or, the solvent used in the preparation method (2) comprises: one or more of ethanol, methanol, and ether;
and/or, the solvents used in the preparation method (3) comprise: Step 1: one or more of methanol, ethanol, isopropanol, and tert-butanol; Step 2: one or more of deuterated methanol and deuterated ethanol; Step 3: one or more of ether and tetrahydrofuran; and Step 4: one or more of n-hexane and petroleum ether.
and/or, the solvent used in the preparation method (4) comprises: one or more of ethanol, methanol, and ether;
and/or, the solvent used in the preparation method (5) comprises: one or more of dichloromethane, toluene, benzene, and pyridine;
and/or, the solvent used in the preparation method (6) comprises: one or more of ethanol, methanol, and ether.
